# FASCICULE DE BREVET EUROPEEN

(11) **EP 2 833 847 B1**
(45) Date de publication et mention de la délivrance du brevet: **08.06.2016**
(21) Numéro de dépôt: 13719961.8
(22) Date de dépôt: 02.04.2013
(51) Int. Cl.: A61F 5/01, A61F 5/14, A43B 7/14

(54) **DISPOSITIF DE MAINTIEN DE LA PARTIE ARRIÈRE DU PIED, ET ORTHÈSE DE CHEVILLE COMPRENANT UN TEL DISPOSITIF**
VORRICHTUNG ZUR BEHANDLUNG DES HINTEREN TEILS DES FUSSES UND KNÖCHELORTHESE MIT EINER SOLCHEN VORRICHTUNG
DEVICE FOR MAINTAINING THE REAR PORTION OF THE FOOT, AND ANKLE ORTHESIS INCLUDING SUCH DEVICE

(30) Priorité: 05.04.2012 FR 1253169
(43) Date de publication de la demande: 11.02.2015
(73) Titulaire: Gibaud, 42000 Saint Etienne (FR)
(72) Inventeur: ANGLADA, Gérard, F-42000 Saint Etienne (FR); GIRARD, Frédéric, F-75009 Paris (FR)
(74) Mandataire: Delorme, Nicolas
(86) Numéro de dépôt international: PCT/FR2013/050717
(87) Numéro de publication internationale: WO 2013/150231

(56) Documents cités:
- WO-A1-2010/070364
- DE-A1-102010 027 418
- FR-A1- 2 930 723
- US-A- 1 575 490
- US-A- 4 686 993
- US-A1- 2005 274 046
- US-A1- 2011 021 963

## Description

La présente invention concerne un dispositif de maintien de la partie arrière du pied, ainsi qu'un orthèse de cheville comprenant un tel dispositif de maintien.

L'entorse de la cheville est un traumatisme qui apparaît lors de la locomotion, pied en appui sur le sol, en particulier :
- lors de l'appui talonnier - c'est-à-dire lors de la phase taligrade de la marche, le poids du corps étant alors sur le talon qui entre en contact avec le sol ;
- et lors de l'appui uni-podal - c'est-à-dire lors de la phase plantigrade, où l'intégralité du poids du corps est sur le pied en charge.

Ainsi, l'instabilité de l'arrière-pied (zone du calcanéum) et du médio-pied (zone du cuboïde et du scaphoïde) lors de la locomotion ou de la course sont particulièrement propices à la survenue d'entorses de la cheville.

Or, la grande majorité des orthèses de cheville existantes n'ont aucune action de stabilisation de l'arrière et du médio-pied par rapport au sol.

En effet, d'une part, on connaît des orthèses comprenant deux coques destinées à enserrer la jambe et reliées par une bande de tissu comportant une portion centrale élargie destinée à être placée sous le pied. Ce type d'orthèse n'offre pas de repère fiable de positionnement pour l'utilisateur, et, de plus, n'assure quasiment aucun maintien du pied.

D'autre part, on connaît des orthèses de cheville comprenant deux coques destinées à enserrer la jambe et une talonnette destinée à être placée sous le pied. Cette talonnette permet de former un repère pour le positionnement du pied dans l'orthèse, mais n'offre que très peu de maintien du pied. Ainsi, elle ne procure pas une stabilisation efficace du pied, et s'avère de plus relativement inconfortable à porter.

Par ailleurs, il n'existe pas de dispositif de maintien de la partie arrière du pied qui puisse être utilisé seul et assurer à lui seul une très bonne stabilisation du pied.

Un dispositif de maintien de la partie arrière du pied selon le préambule de la revendication 1 annexée est connu, par exemple, du document US 1,575,490.

La présente invention vise à remédier aux inconvénients mentionnés ci-dessus.

A cet effet, et selon un premier aspect, l'invention concerne un dispositif de maintien de la partie arrière du pied d'un utilisateur, présentant un plan de symétrie longitudinal et comprenant :
- une semelle sur laquelle la partie arrière du pied est destinée à reposer ;
- deux ailes latérales et un rebord arrière qui s'étendent vers le haut depuis le bord périphérique de la semelle, en position d'utilisation, et qui sont destinés à entourer la partie arrière du pied ;
la semelle possédant :
- un orifice ménagé dans sa partie arrière, et disposé de sorte que, en position d'utilisation, le talon de l'utilisateur puisse s'y loger ;
- un bossage situé sur sa face supérieure, à l'avant de l'orifice, ledit bossage étant symétrique par rapport au plan de symétrie longitudinal ;
de sorte que, en position d'utilisation, le calcanéum du pied de l'utilisateur est sensiblement enclavé dans le dispositif de maintien.

Ainsi, l'invention propose un dispositif de maintien de la partie arrière du pied - en particulier le calcanéum - mais également, au moins en partie, de la partie médiane du pied - en particulier au niveau du scaphoïde et du cuboïde.

En effet, le calcanéum est logé dans l'orifice de la semelle, et maintenu entre le rebord arrière, les ailes latérales, et le bossage. De ce fait, on obtient un excellent maintien, se traduisant par une stabilité latérale (évitant les inclinaisons en valgus ou varus du talon) mais également par l'empêchement du glissement du pied vers l'avant (mouvement pathologique de « tiroir » antéro-postérieur de l'articulation tibio-tarsienne).

Il est à noter que le bossage ne vise pas à combler la voûte du pied mais à former une butée vers l'avant pour le calcanéum, de façon à empêcher le glissement du pied vers l'avant. Toutefois, le bossage peut venir se loger au moins en partie dans l'une des voûtes du pied.

Le dispositif de maintien selon l'invention fournit donc un excellent enclavement du talon, qui permet à la fois de garantir le bon positionnement du pied dans le dispositif mais également la stabilisation du pied dans le dispositif, en position neutre du calcanéum.

Un autre avantage de l'invention réside dans la présence de l'orifice dans la semelle. Celui-ci permet de créer un repère de positionnement facile à utiliser par l'utilisateur lors de la mise en place, et contribue au maintien en améliorant l'effet d'emprisonnement calcanéen. De plus, du fait de cet orifice, le pied équipé du dispositif de maintien n'est pas surélevé par rapport à l'autre pied, évitant ainsi de générer une bascule de bassin et de perturber l'équilibre postural de l'utilisateur. Ceci se traduit également par un confort de port accru.

On note également que le dispositif de maintien selon l'invention est peu encombrant et peut donc se glisser seul dans une chaussure de ville, voire dans une chaussette. De par sa praticité d'utilisation et la gêne réduite qu'il occasionne, ce dispositif de maintien peut plus facilement être adopté et porté régulièrement par un utilisateur.

La longueur de la semelle est par exemple sensiblement comprise entre le tiers et la moitié de la longueur du pied, de façon que le dispositif puisse également permettre la stabilisation de la partie médiane du pied. En effet, la semelle s'étend alors jusqu'au droit du scaphoïde et du cuboïde.

Avantageusement, chaque aile peut s'étendre sur sensiblement l'intégralité du bord latéral correspondant de la semelle et posséder :
- une partie arrière de hauteur suffisante pour que son bord supérieur soit situé au-dessus du bord inférieur du calcanéum, en position d'utilisation ;
- et une partie avant qui, en position d'utilisation, est située sensiblement en vis-à-vis du cuboïde, selon la direction transversale, la partie avant possédant une hauteur qui est supérieure à celle de la partie arrière et qui est suffisante pour que son bord supérieur soit situé au-dessus du bord inférieur du cuboïde.

On peut prévoir que le bord supérieur de la partie arrière de l'aile soit situé au-dessus du bord inférieur du calcanéum en tout point de cette partie arrière.

En outre, le bossage peut être symétrique par rapport à un plan transversal. Par ailleurs, son extrémité arrière peut être adjacente à l'extrémité avant de l'orifice, afin d'améliorer encore l'emprisonnement du talon.

Selon un deuxième aspect, l'invention concerne une orthèse de cheville qui comprend :
- au moins une coque destinée à être placée contre une face latérale de la jambe d'un utilisateur ;
- un support comportant une base destinée à être placée sous le pied et au moins un montant monté sur la coque de façon pivotante autour d'un axe de pivotement sensiblement transversal qui, en position d'utilisation, est sensiblement confondu avec l'axe d'articulation de la cheville ;
- et un dispositif de maintien tel que précédemment décrit, ledit dispositif de maintien étant assemblé à la face supérieure de la base du support.

Du fait de la présence du dispositif de maintien selon l'invention, l'orthèse offre une facilité de positionnement et une stabilisation accrues par rapport aux orthèses de l'art antérieur. L'enclavement talonnier obtenu grâce au dispositif de maintien présente également l'avantage qu'il n'est pas nécessaire de beaucoup serrer l'orthèse pour obtenir un très bon effet de maintien et d'antalgie.

Il est à noter que, de préférence, les ailes du dispositif de maintien sont dépourvues de moyens de fixation ou de pivotement destinés à coopérer avec les coques. En effet, l'articulation de se fait pas sur les ailes du dispositif de maintien mais sur le ou les montants du support.

Selon une réalisation possible, l'orthèse comprend deux coques destinées à être placées l'une contre la face externe et l'autre contre la face interne de la jambe de l'utilisateur, le support comportant deux montants qui, avec la base, forment un U, chacun des montants étant monté sur l'une des coques de façon pivotante autour d'un même axe de pivotement sensiblement transversal.

Le dispositif de maintien et le support peuvent comprendre des moyens d'assemblage mutuel qui sont agencés pour que la position longitudinale du dispositif de maintien par rapport au support puisse être réglée. Ce réglage, qui se fait dans une certaine plage, généralement de façon discrète, permet une meilleure adaptation de l'orthèse à toutes les morphologies.

Les moyens d'assemblage mutuel comprennent par exemple d'une part des nervures et d'autre part des rainures ménagées les unes sur le dispositif de maintien et les autres sur le support, les nervures pouvant être engagées dans les rainures en différentes positions longitudinales.

Afin d'améliorer la respirabilité de l'orthèse, on peut prévoir que la ou chaque coque comprenne :
- une partie externe sensiblement rigide présentant une section transversale courbe dont la concavité est dirigée vers la jambe de l'utilisateur, en position montée ;
- et un capitonnage intérieur monté de façon tendue sur la partie externe, afin de créer une zone de vacuité entre la partie externe et le capitonnage.

On décrit à présent, à titre d'exemples non limitatifs, plusieurs modes de réalisation possibles de l'invention, en référence aux figures annexées :
La figure 1 est une vue en perspective, de côté, d'un dispositif de maintien selon un premier mode de réalisation de l'invention ;
La figure 2 est une vue en perspective, depuis l'arrière, du dispositif de maintien de la figure 1 ;
La figure 3 est une vue en perspective du dispositif de maintien de la figure 1, coupé selon la ligne AA ;
La figure 4 est une vue en section du dispositif de maintien de la figure 1 selon la ligne BB ;
Les figures 5 à 7 sont des vues en perspective d'un pied équipé du dispositif de maintien de la figure 1, respectivement du dessous, depuis l'intérieur et depuis l'extérieur ;
Les figures 8 et 9 sont des représentations schématiques de profil d'un pied équipé du dispositif de maintien de la figure 1, respectivement depuis l'extérieur et depuis l'intérieur ;
Les figures 10 et 11 sont des vues en perspective d'un pied équipé d'une orthèse comprenant un dispositif de maintien selon un deuxième mode de réalisation de l'invention, respectivement vu de côté et vu de dessous ;
La figure 12 est une vue en perspective du dessous d'un dispositif de maintien selon un deuxième mode de réalisation de l'invention ;
La figure 13 est une vue en perspective du dispositif de maintien de la figure 12 assemblé à un support de l'orthèse ;
Les figures 14 à 16 montrent un pied équipé de l'orthèse de la figure 10, respectivement du côté intérieur, de derrière, et du côté extérieur ;
La figure 17 montre schématiquement et partiellement l'orthèse mise en place sur un pied ;
La figure 18 est une vue en perspective du support de l'orthèse ;
Les figures 19 et 20 illustrent le support de la figure 18 assemblé au dispositif de maintien de la figure 12 en deux positions longitudinales distinctes ;
Les figures 21 a et 21 b, 22a et 22b, 23a et 23b sont des vues d'un pied équipé de l'orthèse, et du support assemblé au dispositif de maintien, respectivement en position intermédiaire, en position arrière et en position avant ;
Les figures 24 et 25 sont des vues schématiques d'un pied équipé de l'orthèse, le pied étant respectivement en flexion et en extension ;
La figure 26 est une vue latérale de l'orthèse équipée de moyens de blocage du pivotement ;
Les figures 27 et 28 sont des vues en coupe de l'orthèse de la figure 26, respectivement selon la ligne CC et selon la ligne DD.

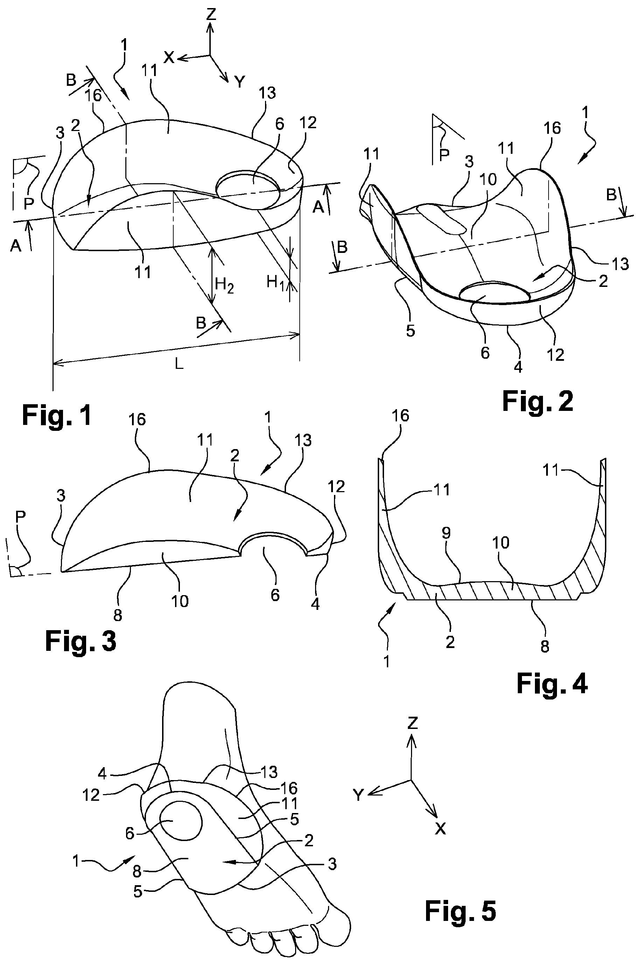

On se réfère tout d'abord aux figures 1 à 9 qui illustrent un dispositif de maintien 1 de la partie arrière du pied d'un utilisateur, selon un premier mode de réalisation.

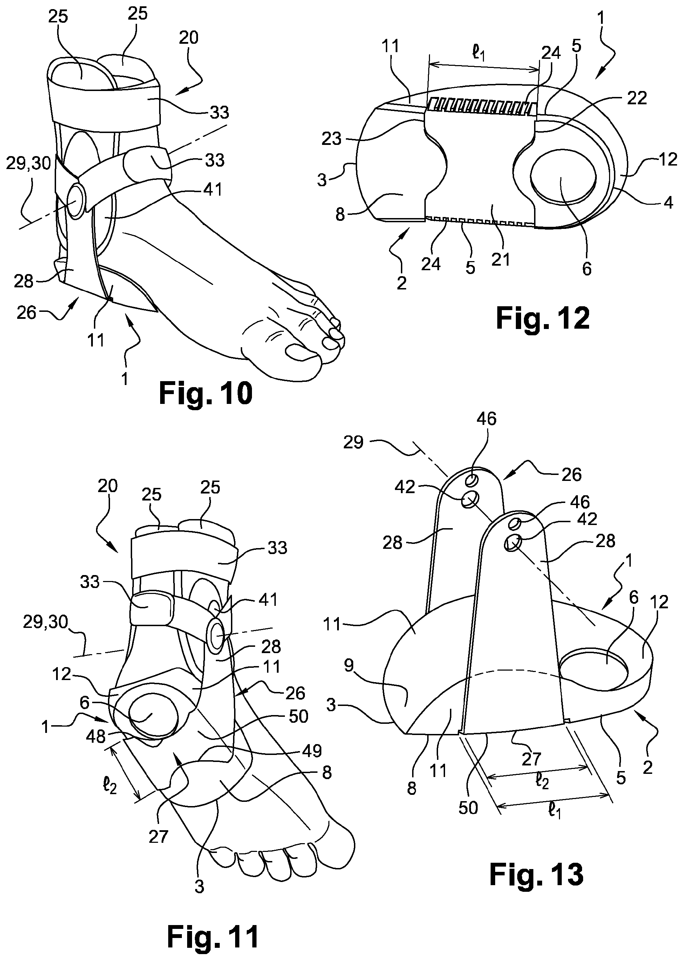

Le dispositif 1, et l'orthèse comprenant un tel dispositif, sont décrits dans la position neutre d'utilisation, c'est-à-dire lorsque l'utilisateur est débout, l'articulation de son pied n'étant ni en flexion ni en extension (comme sur la figure 10).

On définit la direction verticale Z, la direction longitudinale X (antéro-postérieure) et la direction transversale Y (interne-externe). Les termes « hauteur », « supérieur », « inférieur » sont employés en référence à la direction Z ; les termes « avant », « arrière », « longueur » sont employés en référence à la direction X ; et les termes « latéral », « intérieur », « extérieur » sont employés en référence à la direction Y.

Le dispositif 1 présente un plan de symétrie longitudinal P, situé dans un plan (X,Z). Le dispositif 1 est de préférence réalisé en un matériau semi rigide, par exemple en un élastomère, tel que le SEBS (copolymère styrène-éthylène-butylène) ou le TPU (thermoplastique élastomère de polyuréthane), par exemple à forte densité. De ce fait, il présente à la fois une rigidité suffisante pour conférer le maintien nécessaire du pied et une certaine souplesse lui permettant d'une part d'offrir un confort de port satisfaisant et d'autre part d'être utilisé indifféremment pour le pied gauche ou pour le pied droit malgré sa symétrie. Le dispositif 1 peut être réalisé d'une seule pièce par moulage.

Le dispositif 1 comprend tout d'abord une semelle 2 sur laquelle la partie arrière du pied est destinée à reposer. La semelle 2 possède généralement une longueur sensiblement comprise entre le tiers et la moitié de la longueur du pied. Le dispositif 1 peut exister en plusieurs tailles, pour une meilleure adaptation à la morphologie du porteur. A titre d'exemple, la longueur L de la semelle peut être de l'ordre de 110 à 130 mm, par exemple 120 mm, selon la taille du dispositif 1.

La semelle 2 possède un bord périphérique possédant un bord avant 3 sensiblement rectiligne ou légèrement convexe, un bord arrière 4 arrondi et deux bords latéraux 5 sensiblement rectilignes joignant les bords avant 3 et arrière 4.

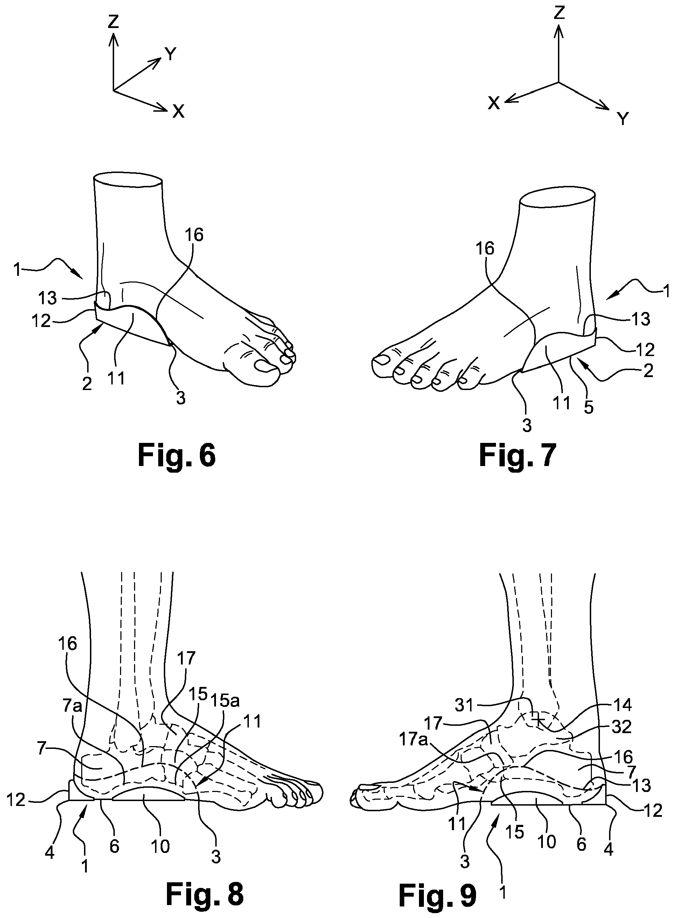

Un orifice 6 est ménagé dans la partie arrière de la semelle 2, l'orifice 6 étant disposé de sorte que, en position d'utilisation, le talon de l'utilisateur puisse s'y loger. En pratique, c'est la arrière et partie inférieure du calcanéum 7 qui vient se loger dans l'orifice 6, comme illustré notamment sur la figure 8. Dans le mode de réalisation représenté, l'orifice 6 est traversant (selon la direction Z) et sensiblement circulaire.

La face inférieure 8 de la semelle 2 peut être sensiblement plane comme on le voit sur la figure 5.

En revanche, la face supérieure 9 de la semelle 2, sur laquelle le pied vient reposer, comprend un bossage 10 ménagé à l'avant de l'orifice 6. Le bossage 10 est symétrique par rapport au plan de symétrie longitudinal P, et peut de plus être symétrique par rapport à un plan transversal, comme on le voit sur la figure 4.

Dans la réalisation représentée, comme on le voit sur la figure 3, l'extrémité arrière du bossage 10 est adjacente à l'extrémité avant de l'orifice 6.

De plus, le dispositif 1 comprend deux ailes latérales 11 et un rebord arrière 12 qui s'étendent vers le haut depuis le bord périphérique de la semelle 2, en position d'utilisation, et qui sont destinés à entourer la partie arrière du pied.

De préférence, chaque aile 11 s'étend sur sensiblement l'intégralité du bord latéral 5 correspondant de la semelle 2 et le rebord arrière 12 joint les deux ailes latérales 11.

Chaque aile 11 peut posséder :
- une partie arrière, par exemple située sensiblement en vis-à-vis de l'orifice 6, de hauteur moyenne H1 suffisante pour que son bord supérieur 13 soit situé au-dessus du bord inférieur 7a du calcanéum 7, en position d'utilisation (voir figure 8) ;
- et une partie avant qui, en position d'utilisation, est située sensiblement en vis-à-vis du cuboïde 15, selon la direction transversale Y. La partie avant possède une hauteur moyenne qui est supérieure à la hauteur H1 de la partie arrière et qui est suffisante pour que son bord supérieur 16 soit situé au-dessus du bord inférieur 15a du cuboïde 15.

Selon une réalisation possible, la hauteur H2 de l'aile latérale 11 à son point le plus haut est au moins de 25 mm, voire au moins de 30 mm. Cette hauteur est suffisamment importante pour permettre le maintien requis, et suffisamment faible pour ne pas constituer une gêne de port notable. Par ailleurs, la hauteur moyenne H1 de la partie arrière de l'aile 11 peut être voisine de la hauteur du rebord arrière 12, par exemple de l'ordre de 10 à 20 mm, par exemple de l'ordre de 15 mm. Les ailes 11 ne sont donc pas de simples bords surélevés, mais bien des moyens de retenue latéraux permettant une augmentation significative de la stabilisation du pied, en partie arrière et en partie médiane.

Ainsi, en position d'utilisation, le calcanéum 7 du pied de l'utilisateur est sensiblement enclavé dans le dispositif de maintien 1, en position neutre - c'est-à-dire dans l'axe physiologique de posture - comme on le voit notamment sur les figures 8 et 9.

En effet, la partie arrière et inférieure du calcanéum 7 est logée dans l'orifice 6. De plus, le calcanéum 7 est bloqué vers l'arrière par le rebord arrière 12, vers l'avant par le bossage 10, et latéralement par les ailes latérales 11 qui présentent avantageusement une hauteur leur permettant d'emprisonner le calcanéum 7.

De plus, les ailes latérales 11 créent une zone de calage sous la scaphoïde 17 (figure 9) et sous le cuboïde 15 pour garantir une stabilisation de l'arche interne et de l'arche externe du pied. Ainsi, il peut avantageusement être prévu que le bord supérieur 16 de la partie avant de l'aile 11 soit situé sensiblement au niveau du bord inférieur 17a du scaphoïde 17 (du côté interne).

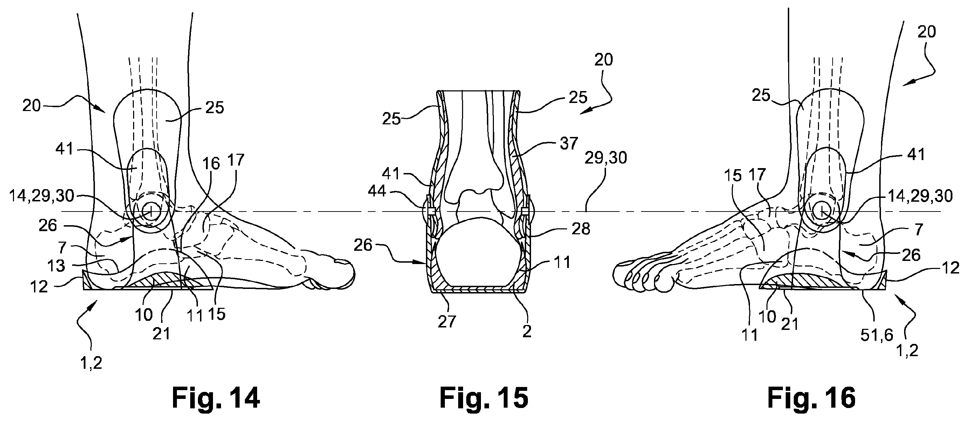

On se reporte à présent aux figures 10 à 28 qui représentent une orthèse 20 comprenant un dispositif de maintien 1 sensiblement identique à celui précédemment décrit.

Cependant, comme cela est visible sur la figure 12, ce dispositif de maintien 1, selon deuxième mode de réalisation, peut se distinguer de celui des figures 1 à 9 par les caractéristiques suivantes.

Tout d'abord, la face inférieure 8 de la semelle 2 peut ne pas être sensiblement plane. Ainsi, le dispositif de maintien 1 peut comporter un canal transversal 21 ouvert vers le bas, en position d'utilisation. Dans la réalisation représentée, ce canal 21 comprend un bord arrière 22 situé à proximité de l'orifice 6 et formant une échancrure concave autour du bord avant de cet orifice 6. Le bord avant 23 peut présenter une forme symétrique, avec une échancrure concave. Toutefois, on peut envisager que les bords 22, 23 soient sensiblement rectilignes et transversaux.

La longueur I1 du canal 21 peut être de l'ordre de 45 à 55 mm, par exemple voisine de 50 mm, et sa hauteur peut être de l'ordre de 2 mm.

De plus, chaque aile latérale 11 du dispositif de maintien 1 peut comporter, au voisinage du bord latéral 5 correspondant et au droit du canal 21, des rainures 24 sensiblement verticales ménagées sur la face extérieure de l'aile 11. Les rainures 24 sont espacées longitudinalement les unes des autres. Leur rôle sera expliqué plus loin.

L'orthèse 20 comprend de plus :
- au moins une coque 25 destinée à être placée contre une face latérale de la jambe d'un utilisateur ;
- un support 26 comportant une base 27 destinée à être placée sous le pied et au moins un montant 28 monté sur la coque 25 de façon pivotante autour d'un axe de pivotement 29 sensiblement transversal qui, en position d'utilisation, est sensiblement confondu avec l'axe d'articulation 30 de la cheville ;
- et un dispositif de maintien 1 tel que précédemment décrit, ledit dispositif de maintien 1 étant assemblé à la face supérieure de la base 27 du support 26.

Comme illustré sur la figure 9, l'axe de l'articulation 30 de la cheville est sensiblement le centre 14 de la poulie astragalienne 31, c'est-à-dire la partie supérieure bombée de l'astragale 32.

Dans la réalisation représentée sur les figures, l'orthèse 20 comporte deux coques 25 destinées à être placées l'une contre la face externe et l'autre contre la face interne de la jambe de l'utilisateur, le support 26 comportant deux montants 28 qui, avec la base 27, forment un U. Chacun des montants 28 est monté sur la coque 25 correspondante de façon pivotante autour du même axe de pivotement 29 sensiblement transversal, correspondant à l'axe de l'articulation 30 de la cheville. Les coques 25 peuvent être identiques.

L'orthèse 20 peut également comporter au moins une sangle 33 apte à serrer la ou chaque coque 25 contre la jambe. Dans la réalisation représentée, l'orthèse 20 comprend une première sangle 33 située sensiblement au niveau de l'articulation de la cheville, qui peut être conçue pour appuyer sur les malléoles, et une deuxième sangle 33 située en partie supérieure des coques 25 et disposée de façon sensiblement circulaire dans un plan horizontal, en position d'utilisation. Les sangles 33 peuvent se refermer sur elles-mêmes autour de la jambe par un système de type Velcro ®.

On décrit à présent plus spécifiquement la coque 25.

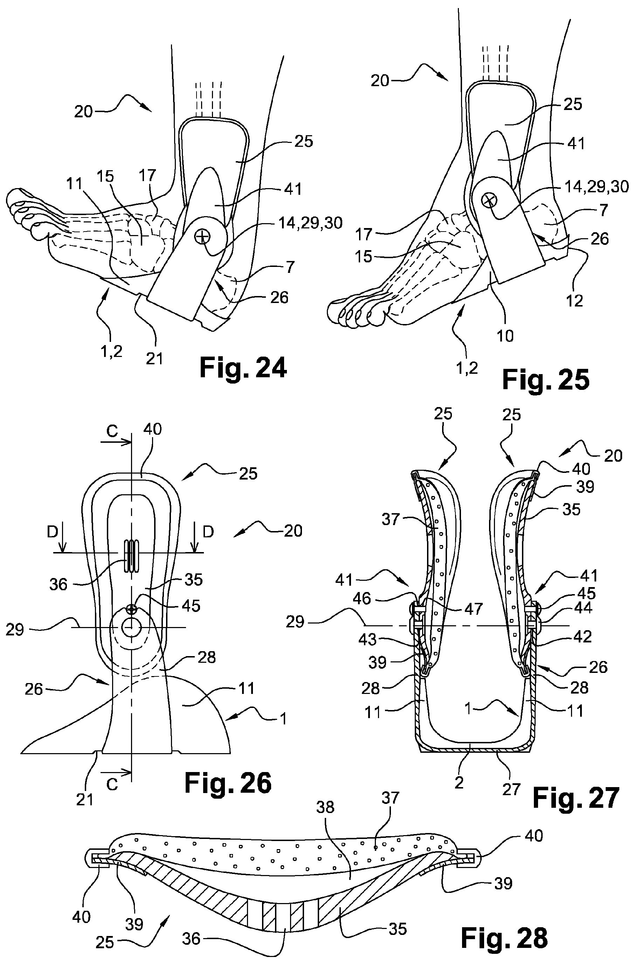

La coque 25 peut présenter une forme allongée verticalement, allant en se rétrécissant du haut vers le bas. Dans la réalisation particulière représentée sur les figures 26 à 28, la coque 25 comporte :
- une partie externe 35 sensiblement rigide, par exemple en polyamide 6,6, qui possède, en vue arrière, sensiblement une forme en S (figure 27) et, en vue de dessus, une forme courbée avec la concavité dirigée vers l'intérieur (figure 28). Cette partie externe 35 peut comporter des lumières 36 de ventilation ;
- un capitonnage intérieur 37 destiné à améliorer le confort de l'utilisateur. Ce capitonnage 37 est constitué d'un coussin de préférence en mousse à haute résilience à structure ouverte, qui laisse passer l'air entre la peau et l'extérieur du coussin. Pour optimiser la respiration de la zone de la jambe équipée de l'orthèse 20, le coussin peut être monté de façon tendue sur la partie externe 35 de la coque 25. Ainsi, une zone 38 de vacuité est formée entre la partie externe 35 et le capitonnage 37 ce qui, en combinaison avec les lumières 36, permet d'augmenter la circulation de l'air entre la peau et l'extérieur. Les risques de macération par transpiration sont donc fortement atténués. Un revêtement de confort et d'hygiène, par exemple en coton, peut être prévu du côté intérieur du capitonnage 37.

Il peut également être prévu une jupe 39 de maintien du capitonnage 37 autour, et du côté externe, de la partie externe 35. Cette jupe 39 peut par exemple être réalisée en néoprène. En outre, la coque 25 peut comporter une bordure textile 40 périphérique enserrant le capitonnage 37 et la jupe 39 pour les maintenir contre la partie externe 35.

Avantageusement, la ou chaque coque 25 peut comprendre un bombement sensiblement ovoïde 41, allongé dans la direction verticale, au niveau de l'axe de pivotement 29, qui est agencé pour pouvoir recevoir la malléole interne ou la malléole externe en position d'utilisation.

La présence d'un bombement présente l'avantage que la coque 25 n'appuie pas sur la pointe de la malléole correspondante, ce qui pourrait gêner l'utilisateur. De plus, cette forme bombée peut permettre de créer un appui périphérique autour de la malléole ce qui est bénéfique en termes de positionnement et de contention.

La forme ovoïde permet d'obtenir des avantages supplémentaires. En effet, grâce à cette forme, et bien que les malléoles externe et interne ne soient pas situées à la même hauteur, une coque 25 peut être placée indifféremment du côté interne ou du côté externe de la jambe de l'utilisateur. Ceci diminue les coûts de fabrication de l'orthèse et simplifie le stockage puisqu'on peut n'avoir qu'un seul type de coque 25. En outre, ceci facilite la mise en place de l'orthèse 20 par l'utilisateur.

On décrit maintenant plus précisément le support 26 et son assemblage aux autres éléments constitutifs de l'orthèse 20.

Le support 26 est par exemple réalisé en polyamide 6,6, éventuellement chargé de fibres de verre.

Ses deux montants 28 présentent chacun, à leur partie supérieure, un trou 42. Les deux trous 42 sont situés à la même hauteur et sont destinés à être placés en regard de trous 43 correspondants ménagés dans les coques 25. Un moyen d'assemblage, tel qu'un rivet 44, est introduit dans les trous 42, 43 correspondants pour former l'axe de pivotement 29 de l'orthèse 20, comme on le voit sur la figure 27.

Les mouvements physiologiques de flexion (figure 24) et extension (figure 25) sont libérés dans la mesure où l'axe de pivotement 29 de l'orthèse 20 et l'axe d'articulation 30 de la cheville sont sensiblement confondus. Ceci peut permettre d'utiliser l'orthèse 20 dans une phase de reprise progressive d'activité.

Toutefois, dans certaines circonstances, il n'est pas souhaitable que l'utilisateur puisse effectuer ces mouvements. Il peut alors être prévu que l'orthèse 20 comprenne des moyens de blocage du pivotement du support 26 par rapport à la ou à chaque coque 25. Le blocage du pivotement peut être obtenu au moyen d'un organe de fixation, tel qu'une vis 45, introduite dans un trou 46 d'un montant 28 du support 26 et un trou 47 de la coque 25 correspondante et situé en regard du trou 46. Comme illustré sur les figures 13, 26 et 27, le trou 46 de blocage peut être situé au-dessus du trou 42 du pivotement.

Quant à la base 27 du support 26, elle peut présenter un bord arrière 48 et un bord avant 49 de forme correspondant respectivement à la forme du bord arrière 22 et du bord avant 23 du canal 21 ménagé dans la semelle 2 du dispositif de maintien 1. Ainsi, les bords arrière 48 et avant 49 de la base 27 du support 26 peuvent présenter des échancrures concaves, comme on le voit sur la figure 11 notamment.

Avantageusement, on peut prévoir que le canal 21 du dispositif de maintien 1 est agencé pour recevoir la base 27 du support 26 de sorte que, en position montée, la face inférieure 50 de la base 27 du support 26 soit située sensiblement dans le même plan que la face inférieure 8 de la semelle 2, ou au-dessus de celle-ci (voir figure 13). Ainsi, il n'y a pas de surépaisseur sous le pied qui nuirait au confort de port.

Le dispositif de maintien 1 et le support 26 peuvent typiquement comprendre des moyens d'assemblage mutuel par emboîtement.

Selon une première réalisation possible, la longueur I2 de la base 27 du support 26 - selon la direction X - est sensiblement identique à la longueur I1 du canal 21. Dans ce cas, la base 27 du support 26 s'encastre sensiblement parfaitement dans le canal 21, les bords avant 23, 49 d'une part et les bords arrière 22, 48 d'autre part étant sensiblement adjacents.

Selon une autre réalisation possible, le dispositif de maintien 1 et le support 26 comprennent des moyens d'assemblage mutuel qui sont agencés pour que la position longitudinale du dispositif de maintien 1 par rapport au support 26 puisse être réglée.

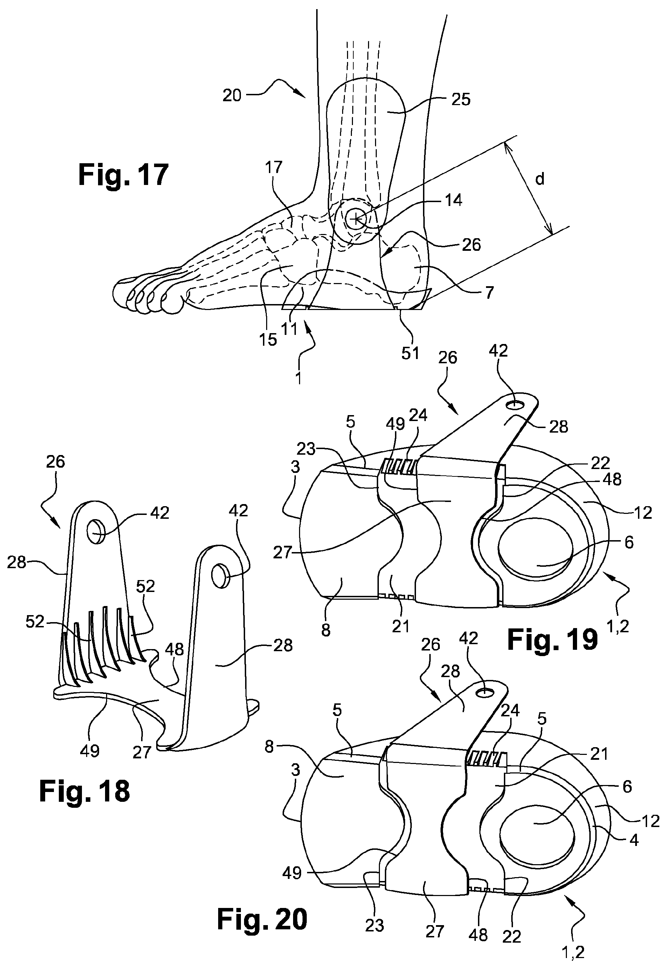

En effet, l'orthèse 20 est d'autant plus efficace et confortable pour l'utilisateur que la distance d entre l'appui calcanéen 51 et le centre 14 de la poulie astragalienne 31 est respecté (voir figure 17). Pour s'adapter à toutes les variations de morphologie et aux différentes tailles de pieds, l'orthèse 20 permet un réglage de cette distance d par déplacement longitudinal du dispositif de maintien 1 par rapport au support 26. En effet, le fait de prévoir des orthèses de tailles différentes (par exemple trois tailles différentes selon la pointure de l'utilisateur) ne suffit pas toujours pour réaliser une adaptation satisfaisante à la morphologie de l'utilisateur, si bien que des gênes pourraient apparaître, comme un frottement sur les malléoles.

De façon concrète, pour régler la distance d, la longueur I2 de la base 27 du support 26 est inférieure à la longueur I1 du canal 21 ménagé dans la semelle 2 du dispositif de maintien 1. Par exemple, la longueur I2 de la base 27 peut être de l'ordre de 35 à 40 mm, par exemple de l'ordre de 37 mm.

Il est prévu sur le support 26, du côté intérieur et à la jonction entre la base 27 et un ou chaque montant 28, une série de nervures 52 s'étendant dans un plan sensiblement transversal, en position d'utilisation (voir figure 18). Ces nervures 52 sont espacées longitudinalement les unes des autres.

Les nervures 52 forment, avec les rainures 24, des moyens d'assemblage mutuel entre le support 26 et le dispositif de maintien 1. Les nervures 52 peuvent être engagées dans les rainures 24 en différentes positions longitudinales. On obtient ainsi un réglage de la position longitudinale du dispositif de maintien 1 par rapport au support 26, selon des valeurs discrètes. Le réglage s'effectue dans une plage délimitée par :
- une position avancée (figure 19), où le bord arrière 48 de la base 27 du support 26 est sensiblement adjacent au bord arrière 22 du canal 21 du dispositif de maintien 1 ;
- et une position reculée, où le bord avant 49 de la base 27 du support 26 est sensiblement adjacent au bord avant 23 du canal 21 du dispositif de maintien 1.

Une fois le positionnement approprié obtenu, le dispositif de maintien 1 est bloqué en translation par rapport à la base 27 du support 26 par emboîtement des nervures 52 du support 26 dans les rainures 24 du dispositif de maintien 1.

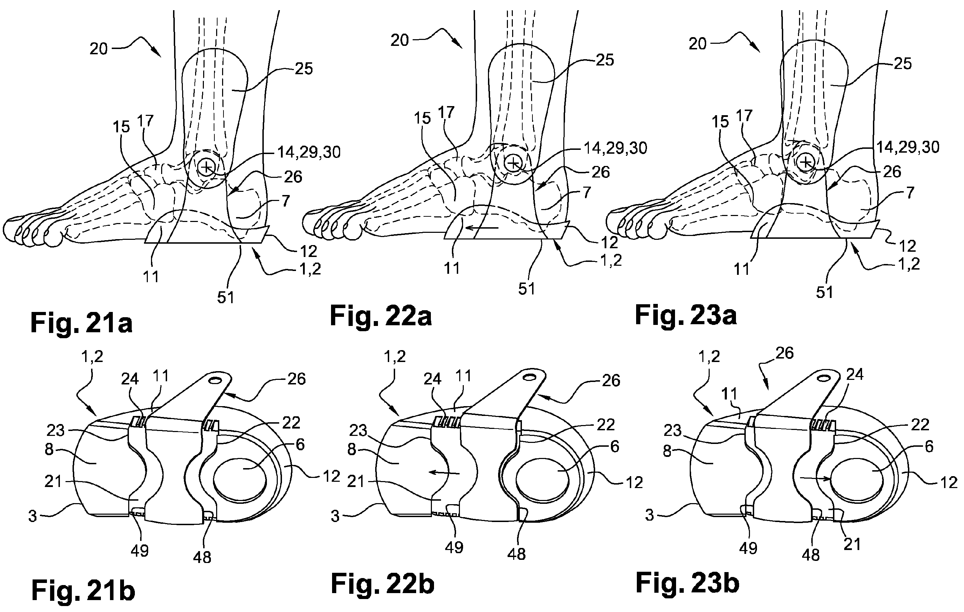

Les figures 21 a à 23b montrent différents positionnements possibles du dispositif de maintien 1 par rapport au support 26.

Sur les figures 21 a et 21 b, il s'agit d'un positionnement sensiblement central, correspondant à la position physiologique normale de l'utilisateur. Les figures 22a et 22b montrent une adaptation à un calcanéum 7 antériorisé, le dispositif de maintien 1 étant en position reculée. Enfin, les figures 23a et 23b montrent une adaptation à un calcanéum 7 postériorisé, le dispositif de maintien 1 étant en position avancée.

Ainsi, l'invention propose un dispositif de maintien et une orthèse de cheville qui stabilisent la marche, en enclavant le calcanéum de la jambe appareillée sans le surélever par rapport au calcanéum de l'autre jambe, et en réduisant considérablement les risques de mise en varus et valgus, ainsi que le coulissement vers l'avant du calcanéum.

Il va de soi que l'invention n'est pas limitée aux modes de réalisation décrits ci-dessus à titre d'exemples mais qu'elle comprend tous les équivalents techniques et les variantes des moyens décrits ainsi que leurs combinaisons.

## Revendications

1. Dispositif de maintien (1) de la partie arrière du pied d'un utilisateur, présentant un plan de symétrie longitudinal (P) et comprenant :
- une semelle (2) sur laquelle la partie arrière du pied est destinée à reposer , la semelle (2) possédant un orifice (6) ménagé dans sa partie arrière, et disposé de sorte que, en position d'utilisation, le talon de l'utilisateur puisse s'y loger ;
**caractérisé en ce que** le dispositif comprend deux ailes latérales (11) et un rebord arrière (12) qui s'étendent vers le haut depuis le bord périphérique de la semelle (2), en position d'utilisation, et qui sont destinés à entourer la partie arrière du pied ; et **en ce que** la semelle (2) possède un bossage (10) situé sur sa face supérieure (9), à l'avant de l'orifice (6), ledit bossage (10) étant symétrique par rapport au plan de symétrie longitudinal (P) ;
de sorte que, en position d'utilisation, le calcanéum (7) du pied de l'utilisateur est sensiblement enclavé dans le dispositif de maintien (1).

2. Dispositif de maintien selon la revendication 1, **caractérisé en ce que** la longueur (L) de la semelle (2) est sensiblement comprise entre le tiers et la moitié de la longueur du pied.

3. Dispositif de maintien selon la revendication 1 ou 2, **caractérisé en ce que** chaque aile (11) s'étend sur sensiblement l'intégralité du bord latéral correspondant de la semelle (2) et possède :
- une partie arrière de hauteur suffisante pour que son bord supérieur (13) soit situé au-dessus du bord inférieur (7a) du calcanéum (7), en position d'utilisation ;
- et une partie avant qui, en position d'utilisation, est située sensiblement en vis-à-vis du cuboïde (15), selon la direction transversale (Y), la partie avant possédant une hauteur qui est supérieure à celle de la partie arrière et qui est suffisante pour que son bord supérieur (16) soit situé au-dessus du bord inférieur (15a) du cuboïde (15).

4. Dispositif de maintien selon l'une des revendications 1 à 3, **caractérisé en ce que** le bossage (10) est symétrique par rapport à un plan transversal.

5. Dispositif de maintien selon l'une des revendications 1 à 4, **caractérisé en ce que** l'extrémité arrière du bossage (10) est adjacente à l'extrémité avant de l'orifice (6).

6. Orthèse de cheville, **caractérisée en ce qu'**elle comprend :
- au moins une coque (25) destinée à être placée contre une face latérale de la jambe d'un utilisateur ;
- un support (26) comportant une base (27) destinée à être placée sous le pied et au moins un montant (28) monté sur la coque (25) de façon pivotante autour d'un axe de pivotement (29) sensiblement transversal qui, en position d'utilisation, est sensiblement confondu avec l'axe d'articulation (30) de la cheville ;
- et un dispositif de maintien (1) selon l'une des revendications 1 à 5, ledit dispositif de maintien (1) étant assemblé à la face supérieure de la base (27) du support (26).

7. Orthèse selon la revendication 6, **caractérisée en ce qu'**elle comprend deux coques (25) destinées à être placées l'une contre la face externe et l'autre contre la face interne de la jambe de l'utilisateur, le support (26) comportant deux montants (28) qui, avec la base (27), forment un U, chacun des montants (28) étant monté sur l'une des coques (25) de façon pivotante autour d'un même axe de pivotement (29) sensiblement transversal.

8. Orthèse selon la revendication 6 ou 7, **caractérisée en ce que** la ou chaque coque (25) comprend un bombement (41) sensiblement ovoïde, allongé dans la direction verticale, au niveau de l'axe de pivotement (29), qui est agencé pour pouvoir recevoir la malléole interne ou la malléole externe en position d'utilisation.

9. Orthèse selon l'une des revendications 6 à 8, **caractérisée en ce que** le dispositif de maintien (1) comprend un canal (21) transversal ouvert vers le bas, en position d'utilisation, qui est agencé pour recevoir la base (27) du support (26) de sorte que, en position montée, la face inférieure (50) de la base (27) du support (26) soit située sensiblement dans le même plan que la face inférieure (8) de la semelle (2) ou au-dessus de celle-ci.

10. Orthèse selon l'une des revendications 6 à 9, **caractérisée en ce que** le dispositif de maintien (1) et le support (26) comprennent des moyens d'assemblage mutuel par emboîtement (24, 52).

11. Orthèse selon l'une des revendications 6 à 10, **caractérisée en ce que** le dispositif de maintien (1) et le support (26) comprennent des moyens d'assemblage mutuel (24, 52) qui sont agencés pour que la position longitudinale du dispositif de maintien (1) par rapport au support (26) puisse être réglée.

12. Orthèse selon la revendication 11, **caractérisée en ce que** les moyens d'assemblage mutuel comprennent d'une part des nervures (52) et d'autre part des rainures (24) ménagées les unes sur le dispositif de maintien (1) et les autres sur le support (26), les nervures (52) pouvant être engagées dans les rainures (24) en différentes positions longitudinales.

13. Orthèse selon l'une des revendications 6 à 12, **caractérisée en ce qu'**elle comprend des moyens de blocage (45) du pivotement du support (26) par rapport à la ou à chaque coque (25).

14. Orthèse selon l'une des revendications 6 à 13, **caractérisée en ce qu'**elle comprend en outre au moins une sangle (33) apte à serrer la ou chaque coque (25) contre la jambe.

15. Orthèse selon l'une des revendications 6 à 14, **caractérisée en ce que** la ou chaque coque (25) comprend :
- une partie externe (35) sensiblement rigide présentant une section transversale courbe dont la concavité est dirigée vers la jambe de l'utilisateur, en position montée ;
- et un capitonnage intérieur (37) monté de façon tendue sur la partie externe (35), afin de créer une zone (38) de vacuité entre la partie externe (35) et le capitonnage (37).

## Patentansprüche

1. Vorrichtung zum Halten (1) des hinteren Teils des Fußes eines Benutzers, die eine Längssymmetrieebene (P) aufweist und Folgendes umfasst:
- eine Sohle (2), auf der der hintere Teil des Fußes aufliegen soll, wobei die Sohle (2) eine Öffnung (6) besitzt, die in ihrem hinteren Teil eingerichtet und derart angeordnet ist, dass sich die Ferse des Benutzers in Gebrauchsposition in sie fügen kann,
**dadurch gekennzeichnet, dass** die Vorrichtung zwei seitliche Flügel (11) und eine hintere Krempe (12) umfasst, die sich in Gebrauchsposition von dem Umfangsrand der Sohle (2) nach oben erstrecken, und die dazu bestimmt sind, den hinteren Teil des Fußes zu umgeben, und dass die Sohle (2) einen Höcker (10) besitzt, der sich auf ihrer Oberseite (9) vor der Öffnung (6) befindet, wobei der Höcker (10) in Bezug zu der Längssymmetrieebene (P) symmetrisch ist,
so dass der Kalkaneus (7) des Fußes des Benutzers in der Gebrauchsposition im Wesentlichen in der Haltevorrichtung (1) eingeschlossen ist.

2. Haltevorrichtung nach Anspruch 1, **dadurch gekennzeichnet, dass** die Länge (L) der Sohle (2) im Wesentlichen zwischen einem Drittel und der Hälfte der Länge des Fußes liegt.

3. Haltevorrichtung nach Anspruch 1 oder 2, **dadurch gekennzeichnet, dass** sich jeder Flügel (11) auf im Wesentlichen dem gesamten seitlichen Rand erstreckt, der der Sohle (2) entspricht, und Folgendes besitzt:
- einen hinteren Teil mit ausreichender Höhe, damit sein oberer Rand (13) in Gebrauchsposition oberhalb des unteren Rands (7a) des Kalkaneus (7) liegt,
- und einen vorderen Teil, der in Gebrauchsposition im Wesentlichen gegenüber dem Kuboid (15) entlang der Querrichtung (Y) liegt, wobei der vordere Teil eine Höhe besitzt, die größer ist als die des hinteren Teils, und die ausreicht, damit sein oberer Rand (16) oberhalb des unteren Rands (15a) des Kuboids (15) liegt.

4. Haltevorrichtung nach einem der Ansprüche 1 bis 3, **dadurch gekennzeichnet, dass** der Höcker (10) in Bezug zu einer Querebene symmetrisch ist.

5. Haltevorrichtung nach einem der Ansprüche 1 bis 4, **dadurch gekennzeichnet, dass** das hintere Ende des Höckers (10) neben dem vorderen Ende der Öffnung (6) liegt.

6. Knöchelorthese, **dadurch gekennzeichnet, dass** sie Folgendes umfasst:
- mindestens eine Schale (25), die dazu bestimmt ist, gegen eine seitliche Fläche des Beins eines Benutzers platziert zu sein,
- einen Träger (26), der eine Basis (47) umfasst, die dazu bestimmt ist, unter dem Fuß platziert zu sein, und mindestens einen Ständer (28), der auf der Schale (25) um eine Schwenkachse (29), die im Wesentlichen quer ist, schwenkend montiert ist, der in Gebrauchsposition im Wesentlichen mit der Gelenkachse (30) des Knöchels zusammenfällt,
- und eine Haltevorrichtung (1) nach einem der Ansprüche 1 bis 5, wobei die Haltevorrichtung (1) auf der Oberseite der Basis (27) des Trägers (26) angebaut ist.

7. Orthese nach Anspruch 6, **dadurch gekennzeichnet, dass** sie zwei Schalen (25) umfasst, die dazu bestimmt sind, eine gegen die Außenseite und die andere gegen die Innenseite des Beins des Benutzers platziert zu werden, wobei der Träger (26) zwei Ständer (28) umfasst, die mit der Basis (47) ein U bilden, wobei jeder der Ständer (48) auf einer der Schalen (25) um ein und dieselbe Schwenkachse (29), die im Wesentlichen quer ist, schwenkend montiert ist.

8. Orthese nach Anspruch 6 oder 7, **dadurch gekennzeichnet, dass** die Schale oder jede Schale (25) eine im Wesentlichen ovoide Wölbung in die vertikale Richtung gestreckt im Bereich der Schwenkachse (29) umfasst, die eingerichtet ist, um den Innenknöchel oder den Außenknöchel in Gebrauchsposition aufzunehmen.

9. Orthese nach einem der Ansprüche 6 bis 8, **dadurch gekennzeichnet, dass** die Haltevorrichtung (1) einen Querkanal (21) umfasst, der in Gebrauchsposition nach unten offen ist, der eingerichtet ist, um die Basis (27) des Trägers (26) derart aufzunehmen, dass die untere Seite (50) der Basis (27) des Trägers (26) in der montierten Position im Wesentlichen in derselben Ebene liegt wie die Unterseite (8) der Sohle (2) oder oberhalb dieser.

10. Orthese nach einem der Ansprüche 6 bis 9, **dadurch gekennzeichnet, dass** die Haltevorrichtung (1) und der Träger (26) Mittel zum gegenseitigen Zusammenbauen durch Einklinken (24, 52) umfassen.

11. Orthese nach einem der Ansprüche 6 bis 10, **dadurch gekennzeichnet, dass** die Haltevorrichtung (1) und der Träger (26) Mittel zum gegenseitigen Zusammenbauen (24, 52) umfassen, die eingerichtet sind, damit die Längsposition der Haltevorrichtung (1) in Bezug zu dem Träger (26) eingestellt werden kann.

12. Orthese nach Anspruch 11, **dadurch gekennzeichnet, dass** die Mittel zum gegenseitigen Zusammenfügen einerseits Rippen (52) und andererseits Nuten (24) umfassen, wobei die einen auf der Haltevorrichtung (1) und die anderen auf dem Träger (26) eingerichtet sind, wobei die Rippen (52) in die Nuten (24) in unterschiedlichen Längspositionen eingefügt werden können.

13. Orthese nach einem der Ansprüche 6 bis 12, **dadurch gekennzeichnet, dass** sie Mittel (45) zum Blockieren des Schwenkens des Trägers (26) in Bezug zu der oder jeder Schale (25) umfasst.

14. Orthese nach einem der Ansprüche 6 bis 13, **dadurch gekennzeichnet, dass** sie außerdem mindestens einen Gurt (33) umfasst, der geeignet ist, die oder jede Schale (25) gegen das Bein zu spannen.

15. Orthese nach einem der Ansprüche 6 bis 14, **dadurch gekennzeichnet, dass** die oder jede Schale (25) Folgendes umfasst:
- einen äußeren Teil (35), der im Wesentlichen starr ist, der einen gebogenen Querschnitt aufweist, dessen Konkavität in montierter Position zu dem Bein des Benutzers gerichtet ist,
- eine Innenpolsterung (37), die gespannt auf dem Außenteil (35) montiert ist, um eine Leerzone (38) zwischen dem Außenteil (35) und der Polsterung (37) zu schaffen.

## Claims

1. A device for holding (1) the rear portion of a user's foot, having a longitudinal plane of symmetry (P) and comprising:
- a sole (2) on which the rear portion of the foot is intended to rest, the sole (2) having a hole (6) arranged in its rear portion, and disposed so that, in the use position, the user's heel can be housed therein;
**characterized in that** the device comprises two side wings (11) and a rear rim (12) which extend upwardly from the peripheral edge of the sole (2), in the use position, and which are intended to surround the rear portion of the foot;
and **in that** the sole (2) has a boss (10) located on its upper face (9), at the front of the hole (6), said boss (10) being symmetrical with respect to the longitudinal plane of symmetry (P);
so that, in the use position, the calcaneum (7) of the user's foot is substantially enclosed in the holding device (1).

2. The holding device according to claim 1, **characterized in that** the length (L) of the sole (2) is substantially comprised between the third and the half of the foot length.

3. The holding device according to claim 1 or 2, **characterized in that** each wing (11) extends substantially over the entire corresponding side edge of the sole (2) and has:
- a rear portion of a sufficient height such that its upper edge (13) is located above the lower edge (7a) of the calcaneum (7), in the use position;
- and a front portion which, in the use position, is substantially located opposite the cuboid (15), in the transverse direction (Y), the front portion having a height which is greater than that of the rear portion and which is sufficient such that its upper edge (16) is located above the lower edge (15a) of the cuboid (15).

4. The holding device according to any of claims 1 to 3, **characterized in that** the boss (10) is symmetrical with respect to a transverse plane.

5. The holding device according to any of claims 1 to 4, **characterized in that** the rear end of the boss (10) is adjacent to the front end of the hole (6).

6. An ankle orthosis, **characterized in that** it comprises:
- at least one shell (25) intended to be placed against a side face of the leg of a user;
- a support (26) including a base (27) intended to be placed under the foot and at least one upright (28) pivotally mounted on the shell (25) about a substantially transverse pivot axis (29) which, in the use position, is substantially coincident with the axis of articulation (30) of the ankle;
- and a holding device (1) according to any of claims 1 to 5, said holding device (1) being assembled to the upper face of the base (27) of the support (26).

7. The orthosis according to claim 6, **characterized in that** it comprises two shells (25) intended to be placed, the one against the outer face, and the other against the inner face of the leg of the user, the support (26) including two uprights (28) which, with the base (27), form a U, each of the uprights (28) being pivotally mounted on one of the shells (25) about the same substantially transverse pivot axis (29).

8. The orthosis according to claim 6 or 7, **characterized in that** the or each shell (25) comprises a substantially ovoid bulge (41), elongated in the vertical direction, at the pivot axis (29), which is arranged to be able to receive the inner malleolus or the outer malleolus in the use position.

9. The orthosis according to any of claims 6 to 8, **characterized in that** the holding device (1) comprises a downwardly open transverse channel (21), in the use position, which is arranged to receive the base (27) of the support (26) so that, in the mounted position, the lower face (50) of the base (27) of the support (26) is substantially located in the same plane as the lower face (8) of the sole (2) or above it.

10. The orthosis according to any of claims 6 to 9, **characterized in that** the holding device (1) and the support (26) comprise mutual assembly means by interlocking (24, 52).

11. The orthosis according to any of claims 6 to 10, **characterized in that** the holding device (1) and the support (26) comprise mutual assembly means (24, 52) which are arranged so that the longitudinal position of the holding device (1) relative to the support (26) can be adjusted.

12. The orthosis according to claim 11, **characterized in that** the mutual assembly means comprise, on the one hand, ribs (52) and, on the one hand, grooves (24) arranged, the ones on the holding device (1), and the others on the support (26), the ribs (52) being engageable in the grooves (24) in different longitudinal positions.

13. The orthosis according to any of claims 6 to 12, **characterized in that** it comprises means for blocking (45) the pivoting of the support (26) relative to the or to each shell (25).

14. An orthosis according to any of claims 6 to 13, **characterized in that** it further comprises at least one strap (33) adapted to clamp the or each shell (25) against the leg.

15. The orthosis according to any of claims 6 to 14, **characterized in that** the or each shell (25) comprises:
- a substantially rigid outer portion (35) having a curved cross section whose concavity is directed towards the user's leg, in the mounted position;
- and an inner cushioning (37) mounted in a tensioned manner on the outer portion (35), in order to create a vacuity area (38) between the outer portion (35) and the cushioning (37).
